# EUROPEAN PATENT APPLICATION

(11) **EP 3 882 631 A1**
(43) Date of publication of application: **22.09.2021**
(21) Application number: 19883201.6
(22) Date of filing: 08.11.2019
(51) Int. Cl.: G01N 33/531, G01N 33/543

(54) **METHOD FOR SUPPRESSING ABNORMAL DETECTION IN IMMUNOASSAY BY AUTOMATIC ANALYSIS DEVICE, AND IMMUNOASSAY REAGENT**

(30) Priority: 09.11.2018 JP 2018211559
(71) Applicant: Sekisui Medical Co., Ltd., Tokyo 103-0027 (JP)
(72) Inventor: YOSHIDA, Tadaaki, Tokyo 103-0027 (JP); MIZUNIWA, Chihiro, Tokyo 103-0027 (JP); KITAHARA, Shinichiro, Tokyo 103-0027 (JP); TAKAHASHI, Hiroshi, Tokyo 103-0027 (JP)
(74) Representative: Hoffmann Eitle
(86) International application number: PCT/JP2019/043799
(87) International publication number: WO 2020/096029

(57) **Abstract**

There is provided a method for preventing an occurrence of an irregular detection value and performing measurement with good reproducibility in an immunoassay using an automatic analyzer regardless of whether the reaction cuvette used is of a disposable type or a reusable type and whether the optical detection method used measures transmitted light or scattered light. There is also provided an immunoassay reagent for use in the method. A method for preventing the occurrence of irregular detection and enhancing the reproducibility of measurement includes using a reagent containing polyethylene glycol or the like having a weight-average molecular weight of 300 to 3000.

## Description

### TECHNICAL FIELD

The present invention relates to a method for preventing irregular detection, which occurs when optically detecting a turbidity change caused by an immunoreaction in an immunoassay using an automatic analyzer, thereby enhancing the measurement reproducibility, and to an immunoassay reagent for use in the method. In particular, the present invention relates to a method for preventing such irregular detection in a latex agglutination immunoassay using an automatic analyzer, thereby enhancing the measurement reproducibility, and to an immunoassay reagent for use in the method.

### BACKGROUND ART

In clinical assays, the concentration of an analyte contained in a specimen is measured, and the measured value is used for early detection of a disease or determination of a therapeutic effect. These days automatic analyzers are widely used in clinical assays in order to perform measurement of a large number of specimens accurately in a short time. A widely-used method for measuring the concentration of an analyte with an automatic analyzer involves mixing a specimen and a reagent for automatic analysis, and optically detecting a concentration-dependent change of the analyte per unit time. Transmitted light or scattered light is generally used for the optical detection.

Reagents for automatic analysis include, for example, a reagent which is based on the principle of latex immunoagglutination assay (also called latex turbidimetric immunoassay, and hereinafter sometimes referred to as LTIA). LTIA is a measuring method which uses, for example, immunoassay particles to which an antibody against an analyte is coupled (hereinafter sometimes referred to as antibody-coupled immunoassay particles), and detects, e.g. by an optical method (e.g. a turbidimetric method which measures transmitted light or a nephelometric method which measures scattered light), a turbidity change caused by agglutination of the antibody-coupled immunoassay particles due to bonding between the analyte, which is an antigen, and the antibody of the antibody-coupled immunoassay particles by an antigen-antibody reaction.

Most of currently used automatic analyzers perform measurement by mixing a specimen and a reagent in a transparent plastic or glass container, called a reaction cuvette, under irradiation with light, and detecting an optical change of transmitted light or scattered light at regular time intervals. Such reaction cuvettes include a disposable-type cuvette which is to be disposed of after measurement, and a reusable-type cuvette which is to be cleaned after measurement and reused. Causative factors for irregular detection are known which hinder optical detection in measurement using either type of automatic analyzer, thereby reducing the accuracy and reproducibility of measurement. The causative factors include bubbles or dirt adhering to a reaction cuvette, poor mixing (nonuniformity of the solution) of a reagent, etc.

As used herein, "irregular detection (abnormality in detection)" includes a case where, in a simultaneous reproducibility test performed by successively measuring specimens, a measurement value which is clearly divergent from the other measurement values is detected, and a case where due to the influence of the divergent value, the CV value (coefficient of variation) of the simultaneous reproducibility test exceeds 10%. In an instance of such a case, in a measurement of a specimen having a known concentration, the ratio (hereinafter referred to as accuracy) of the measured concentration to the known concentration falls outside the range of 80 to 120%, or even the accuracy of the measurement value is greatly influenced and falls outside the range of 50 to 150%.

### CITATION LIST

### PATENT LITERATURE

Patent Document 1: Japanese Patent Laid-Open Publication No. 2013-140035
Patent Document 2: Japanese Patent Laid-Open Publication No. 2013-068443
Patent Document 3: Japanese Patent Laid-Open Publication No. 2003-226893
Patent Document 4: International Publication No. WO 2011/065573 Pamphlet
Patent Document 5: Japanese Patent Laid-Open Publication No. S58-047256
Patent Document 6: Japanese Patent Laid-Open Publication No. 2003-066047

### SUMMARY OF INVENTION

### TECHNICAL PROBLEM

Patent Document 1, for example, has reported the use of an immunoassay reagent containing a surfactant as a method for preventing the generation of bubbles that adhere to a disposable-type reaction cuvette. This document discloses a method which involves performing a reaction and/or measurement in the presence of a non-ionic polyoxyethylene surfactant, thereby preventing attachment of bubbles onto the side surface of a dried cuvette.

However, when a surfactant is present in an immunoassay system, the surfactant, depending on the type, can inhibit an antigen-antibody reaction, or dissociate aggregates that have been formed by the antigen-antibody reaction. Thus, even when an appropriate surfactant is selected, there is still a possibility of a decrease in measurement sensitivity. Furthermore, the surfactant can sometimes cause, in a complex manner, interference with the immunoassay system, such as a structural change of an analyte itself, the formation of a complex with the analyte, non-specific adsorption onto antibody-coupled latex particles, and detaching the antibody and the blocking protein from the latex particles.

Patent Document 2, for example, has proposed an automatic analyzer which performs a periodic and automatic check for the state of an assay section, including dirt of a reusable-type reaction cuvette, thereby enhancing the accuracy of measurement data.

Patent Document 3, for example, has proposed a reaction cuvette-cleaning solution containing a particular organic solvent for removal of dirt from a reaction cuvette especially after the use of an LTIA reagent.

Patent Document 4, for example, has proposed a method for preventing poor mixing of a reagent (nonuniformity of the solution). The method involves adding a silicone antifoam to an immunoassay reagent, and can improve the accuracy of measurement for various automatic analyzers using different stirring/mixing processes.

On the other hand, it is known that the use of PEG (polyethylene glycol) in an LTIA reagent produces an effect of promoting agglutination of antibody-coupled immunoassay particles, thereby intensifying the signal, as disclosed e.g. in Patent Document 5 and Patent Document 6. Patent Document 5 teaches that the use of PEG can increase the rate of change in the intensity of transmitted light or scattered light (promotion of agglutination), thereby intensifying the signal and enabling highly-sensitive, highly-accurate measurement, and also teaches that the effect increases with increase in the average molecular weight of polyethylene glycol. In fact, in working examples of Patent Document 5, there is no significant difference in the effect between the use of PEG having an average molecular weight of 1540 (PEG 1540) or 4000 (PEG 4000) and the control (without PEG). On the other hand, the use of PEG having an average molecular weight of not less than 6000 produces a high effect, and PEG 6000 is used in all the other examples. Patent Document 6 also teaches that PEG 6000 having an average molecular weight of 6000 is widely used for promotion of an immunoagglutination reaction and highly-sensitive measurement. Thus, the use of PEG in immunoagglutination assay is limited to the use of PEG having a relatively high molecular weight for the purpose of increasing the sensitivity, thereby enabling highly-sensitive, highly-accurate measurement.

However, it is concerned that the strong sensitivity-enhancing effect of PEG having a relatively high average molecular weight of 6000 as disclosed in patent documents 5 and 6, can affect a measurement value. Further, the measurement methods of Patent Document 5 and Patent Document 6 are manual methods, and these documents are silent as to the prevention of irregular detection and the improvement of measurement reproducibility upon measurement with an automatic analyzer.

As will be appreciated from the foregoing, some irregularity or abnormality in detection is still difficult to avoid in an immunoassay using an automatic analyzer, and the above-described techniques cannot fully solve the problem of measurement reproducibility.

In view of the current situation described above, it is therefore an object of the present invention to provide a method for preventing the occurrence of irregular detection and performing measurement with good reproducibility in an immunoassay using an automatic analyzer regardless of whether the reaction cuvette used is of the disposable type or the reusable type and whether the optical detection method used measures transmitted light or scattered light, and to provide an immunoassay reagent for use in the method.

### SOLUTION TO PROBLEM

The present inventors, through intensive studies, have found that the use of polyethylene glycol or the like ("polyethylene glycol" is hereinafter sometimes referred to as "PEG", and "polyethylene glycol or the like" as "PEG or the like"), having a particular average molecular weight (weight-average molecular weight as described below), in an immunoassay reagent does not cause an alteration in the measurement sensitivity, and can prevent irregular detection and enhance the reproducibility of measurement.

Thus, the present invention relates to the following items (1) to (17).
(1) A method for preventing an occurrence of an irregular detection value and enhancing reproducibility of measurement upon optical detection of a reaction between an analyte in a specimen and an immunoassay reagent with an automatic analyzer, wherein the immunoassay reagent contains polyethylene glycol or the like having a weight-average molecular weight of 300 to 3000.
(2) The method according to item (1), wherein the polyethylene glycol or the like is at least one member selected from the group consisting of polyethylene glycol, polypropylene glycol, polybutylene glycol, polyoxyethylene polyoxypropylene glycol, and polyglycerol.
(3) The method according to item (1) or (2), wherein the concentration of the polyethylene glycol or the like in the reaction system is 0.05 to 0.5%.
(4) The method according to any one of items (1) to (3), wherein the polyethylene glycol or the like is polyethylene glycol.
(5) The method according to any one of items (1) to (4), wherein the immunoassay reagent is a reagent for latex turbidimetric immunoassay.
(6) A method for optically detecting a reaction between an analyte in a specimen and an immunoassay reagent with an automatic analyzer, wherein the immunoassay reagent contains polyethylene glycol or the like having a weight-average molecular weight of 300 to 3000.
(7) The method according to item (6), wherein the polyethylene glycol or the like is at least one member selected from the group consisting of polyethylene glycol, polypropylene glycol, polybutylene glycol, polyoxyethylene polyoxypropylene glycol, and polyglycerol.
(8) The method according to item (6) or (7), wherein the concentration of the polyethylene glycol or the like in the reaction system is 0.05 to 0.5%.
(9) The method according to any one of items (6) to (8), wherein the polyethylene glycol or the like is polyethylene glycol.
(10) The method according to any one of items (6) to (9), wherein the immunoassay reagent is a reagent for latex turbidimetric immunoassay.
(11) An immunoassay reagent for use in an optical detection method using an automatic analyzer, the reagent comprising polyethylene glycol or the like having a weight-average molecular weight of 300 to 3000.
(12) The immunoassay reagent according to item (11), wherein the polyethylene glycol or the like is at least one member selected from the group consisting of polyethylene glycol, polypropylene glycol, polybutylene glycol, polyoxyethylene polyoxypropylene glycol, and polyglycerol.
(13) The immunoassay reagent according to item (11) or (12), wherein the reagent is adjusted so that the concentration of the polyethylene glycol or the like in the reaction system becomes 0.05 to 0.5%.
(14) The immunoassay reagent according to any one of items (11) to (13), wherein the polyethylene glycol or the like is polyethylene glycol.
(15) The immunoassay reagent according to any one of items (11) to (14), wherein the immunoassay reagent is a reagent for latex turbidimetric immunoassay.
(16) The immunoassay reagent according to item (15), wherein the reagent comprises a first reagent containing a buffer, and a second reagent containing a latex, and wherein one or both of the first and second reagents contain the polyethylene glycol or the like having a weight-average molecular weight of 300 to 3000.
(17) An immunoassay reagent kit comprising the immunoassay reagent according to any one of items (11) to (16).
   The above-described methods (1) to (10) can also be expressed as follows.
(18) A method for preventing the occurrence of an irregular detection value and enhancing the reproducibility of measurement upon optical detection of a reaction between an analyte in a specimen and an immunoassay reagent with an automatic analyzer, the method comprising a step of bringing the analyte in the specimen into contact with the immunoassay reagent in the presence of polyethylene glycol or the like having a weight-average molecular weight of 300 to 3000.
(19) The method according to item (18), wherein the polyethylene glycol or the like is at least one member selected from the group consisting of polyethylene glycol, polypropylene glycol, polybutylene glycol, polyoxyethylene polyoxypropylene glycol, and polyglycerol.
(20) The method according to item (18) or (19), wherein the concentration of the polyethylene glycol or the like in the reaction system is 0.05 to 0.5%.
(21) The method according to any one of items (18) to (20), wherein the polyethylene glycol or the like is polyethylene glycol.
(22) The method according to any one of items (18) to (21), wherein the immunoassay reagent is a reagent for latex turbidimetric immunoassay.
(23) A method for optically detecting a reaction between an analyte in a specimen and an immunoassay reagent with an automatic analyzer, the method comprising a step of bringing the analyte in the specimen into contact with the immunoassay reagent in the presence of polyethylene glycol or the like having a weight-average molecular weight of 300 to 3000.
(24) The method according to item (23), wherein the polyethylene glycol or the like is at least one member selected from the group consisting of polyethylene glycol, polypropylene glycol, polybutylene glycol, polyoxyethylene polyoxypropylene glycol, and polyglycerol.
(25) The method according to item (23) or (24), wherein the concentration of the polyethylene glycol or the like in the reaction system is 0.05 to 0.5%.
(26) The method according to any one of items (23) to (25), wherein the polyethylene glycol or the like is polyethylene glycol.
(27) The method according to any one of items (23) to (26), wherein the immunoassay reagent is a reagent for latex turbidimetric immunoassay.

### ADVANTAGEOUS EFFECTS OF INVENTION

According to the present invention, by using PEG or the like having a particular average molecular weight in an immunoassay reagent for use in an automatic analyzer, it becomes possible to provide a method for preventing the occurrence of irregular detection and enhancing the reproducibility of measurement without causing a decrease in the measurement sensitivity. It thus becomes possible to enhance the accuracy and reproducibility of immunoassay in an automatic analyzer.

### BRIEF DESCRIPTION OF THE DRAWING

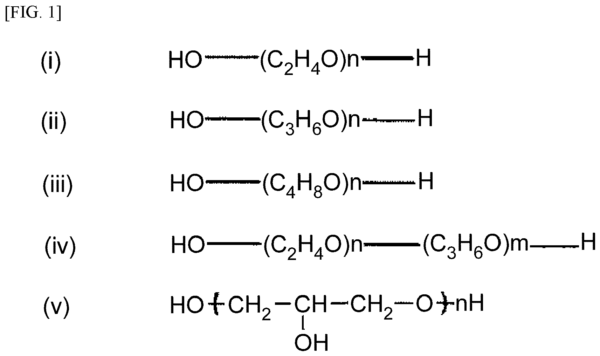

FIG. 1 shows the chemical structures of typical PEGs or the like: (i) polyethylene glycol; (ii) polypropylene glycol; (iii) polybutylene glycol; (iv) polyoxyethylene polyoxypropylene glycol; and (v) polyglycerol.

### DESCRIPTION OF EMBODIMENTS

The present invention will now be described. In the following description, content percentages of PEG or the like are by mass (W/V %) unless otherwise indicated.

### (Immunoassay Reagent)

The immunoassay reagent (reagent solution) according to the present invention contains, besides a main reaction component, the below-described PEG or the like having a particular weight-average molecular weight.

The immunoassay reagent may contain a buffer, such as acetate buffer, citrate buffer, phosphate buffer, Tris buffer, glycine buffer, borate buffer, carbonate buffer, a Good's buffer, or a sodium, potassium or calcium salt thereof, as a component for buffering or adjusting the pH, the ion intensity, the osmotic pressure, etc. of a sample. The immunoassay reagent may also contain a polymer, such as polyvinylpyrrolidone or a phospholipid polymer, as a component for promoting agglutination. Further, the immunoassay reagent may contain, as a component for controlling agglutination, a commonly-used material(s) such as a protein(s), an amino acid(s), a sugar(s), a metal salt(s), a surfactant(s), a reducing substance(s), a chaotropic substance(s), etc., either singly or in a combination of two or more.

The present invention relates to a method for optically detecting a reaction between an analyte in a specimen and the immunoassay reagent with an automatic analyzer, or a method for preventing the occurrence of irregular detection and enhancing the reproducibility of measurement in the optical detection method. Thus, the present invention relates to such a method comprising a step of bringing an analyte in a specimen into contact with the immunoassay reagent in the presence of PEG or the like having a particular weight-average molecular weight.

### (Immunoassay Particles)

Immunoassay particles for use in the present invention may be any known particles as long as they can support a coupling partner specific for the target component of interest. Latex particles using a polymer material such as polystyrene can be preferably used; however, depending on the method for supporting a coupling partner specific for an analyte, inorganic particles such as a metal colloid, silica or carbon can also be used as immunoassay particles according to the present invention. The size of the immunoassay particles can be appropriately selected from the range of 0.05 to 1 µm so that the intended measurement sensitivity, measurement range, etc. can be obtained in consideration of the optical measurement method used (e.g., a turbidimetric method which measures transmitted light or a nephelometric method which measures scattered light). An average particle size of 0.1 to 0.4 µm is generally used in optical detection in automatic analyzers; however, the size of the immunoassay particles is not limited to this range.

The average particle size of the immunoassay particles can be determined, for example, by a particle size analyzer or with transmission electron microscope images. The concentration of immunoassay particles in a reagent can be appropriately selected from, for example, the range of 0.0001 mg/mL to 10 mg/mL according to the particle size of the immunoassay particles used and the design of the entire measurement system.

When latex particles are employed as immunoassay particles in the present invention, no particular limitation is placed on the synthetic polymer that constitutes the latex particles. Examples of usable polymers include polystyrene, a styrene-styrenesulfonate copolymer, polymethacrylic acid, polyacrylic acid, polyitaconic acid, and a styrene-hydrophilic carboxy monomer copolymer such as a styrene-methacrylic acid copolymer, a styrene-acrylic acid copolymer or a styrene-itaconic acid copolymer. Among them, a styrene-methacrylic acid copolymer, a styrene-itaconic acid copolymer, styrene and a styrene-styrenesulfonate copolymer are preferred, and styrene and a styrene-(meth)acrylic acid copolymer are especially preferred.

Examples of the salt of styrenesulfonate include, and are not limited to, sodium salt, potassium salt, lithium salt, and ammonium salt. These salts may be used singly or in a combination of two or more. Among them, sodium styrenesulfonate is especially preferred.

Methacrylic acid, acrylic acid, itaconic acid, maleic acid, fumaric acid, etc. can be used as the hydrophilic carboxy monomer for use in the present invention. Among them, methacrylic acid and acrylic acid are preferred.

### (Assay Specimen)

Examples of an analyte-containing specimen for use in the present invention include blood, serum, plasma, culture supernatant, urine, spinal fluid, saliva, sweat, and ascites of a human or animal, and an extract from the cell or tissue.

The immunoassay reagent of the present invention can be used for measurement of a variety of substances (analytes) contained in an assay specimen. Such analytes include a protein, a peptide, an amino acid, a lipid, a carbohydrate, a nucleic acid, a hapten, etc. No particular limitation is placed on an analyte as long as it is theoretically measurable. Specific examples of analytes may include CRP (C reactive protein), Lp(a), MMP 3 (matrix metalloproteinase 3), anti-CCP (cyclic citrullinated peptide) antibody, anti-phospholipid antibody, RPR, type IV collagen, PSA, BNP (brain natriuretic peptide), NT-proBNP, insulin, microalbumin, cystatin C, RF (rheumatoid factor), CA-RF, KL-6, PIVKA-II, FDP, D-dimer, SF (soluble fibrin), TAT (thrombin-antithrombin III complex), PIC, PAI, factor XIII, pepsinogen I, pepsinogen II, phenytoin, phenobarbital, carbamazepine, valproic acid, theophylline, etc.

The immunoassay reagent of the present invention is composed of a single reagent solution, or a plurality of, i.e. two or more, reagent solutions. The plurality of reagent solutions may include, for example, a reagent solution consisting of a buffer solution e.g. for adjusting an analyte to a concentration suitable for measurement or for adjusting an environment for an antigen-antibody reaction, and a reagent solution containing antibody-coupled particles. The PEG or the like of the present invention may be contained in all the reagent solutions constituting the immunoassay reagent, or contained selectively in one of the reagent solutions. In the case of an immunoassay reagent using latex particles, an exemplary reagent comprises a first reagent containing a buffer and a second reagent containing a latex, and one or both of the reagents contain polyethylene glycol or the like having a weight-average molecular weight of 300 to 3000.

The kit of the present invention, comprising the immunoassay reagent, may include other components necessary for measurement, such as a cleaning liquid, a specimen diluent, a specimen extraction liquid, a sampling pipette, a standard preparation, an instruction manual, etc.

### (PEG or the like)

No particular limitation is placed on the PEG or the like for use in the present invention as long as it has the effect of preventing irregular detection. Typical PEGs or the like are as follows (see FIG. 1).
- Polyethylene glycol (i)
- Polypropylene glycol (ii)
- Polybutylene glycol (iii)
- Polyoxyethylene polyoxypropylene glycol (iv)
- Polyglycerol (v)

In the Figure, m and n each represent a repeated structure, and may each be any value that satisfies the below-described range of weight-average molecular weight. When a polyethylene chain and a polypropylene chain are co-present, they may be distributed at random. While Polyethylene glycol (PEG) is preferably used, any mixture of the above materials can also be used.

These PEGs or the like can be exemplified by the following commercial products. Examples of polyethylene glycol include polyethylene glycol (Kishida Chemical), ADEKA PEG (ADEKA), and PEG (Sanyo Chemical Industries). Examples of polypropylene glycol include UNIOL (registered trademark) D (NOF) and Newpole (registered trademark) PP (Sanyo Chemical Industries). An example of polybutylene glycol is UNIOL (registered trademark) PB (NOF). Examples of polyoxyethylene polyoxypropylene glycol include Emulgen (registered trademark) PP-290 (KAO), PRONON (registered trademark) (NOF), ADEKA (registered trademark) Pulronic L/P/F (ADEKA), and Newpole (registered trademark) PE (Sanyo Chemical Industries). An example of polyglycerol is POLYGLYCERIN (Sakamoto Yakuhin Kogyo).

The effect of preventing irregular detection is determined by the weight-average molecular weight of the PEG or the like, as described in detail in the following examples.

The weight-average molecular weight of the PEG or the like according the present invention is preferably not more than 3000 because if it is too high, the effect of enhancing sensitivity will be high, resulting in a significant change in a measurement value *per se.* The upper limit is preferably 3000 or lower, more preferably 2800 or lower, even more preferably 2500 or lower, and most preferably 2100 or lower.

On the other hand, the weight-average molecular weight of the PEG or the like is preferably not less than 300 because if it is too low, no appreciable irregular detection-preventing effect will be produced. The lower limit is preferably 300 or higher, more preferably 400 or higher, even more preferably 1000 or higher, and most preferably 1900 or higher.

Thus, the range of the weight-average molecular weight of the PEG or the like for use in the present invention is a range which can exert both the sensitivity-enhancing effect and the irregular detection-preventing effect of the PEG or the like, and which was discovered by the present invention. The range of the weight-average molecular weight of the PEG or the like according to the present invention is preferably 300 to 3000, more preferably 400 to 2500, even more preferably 400 to 2100, and most preferably 1900 to 2100.

The concentration of the PEG or the like for use in the present invention in the assay reaction system may be one which ensures the intended irregular detection-preventing effect. In general, as with the weight-average molecular weight, the sensitivity-enhancing effect is high when the concentration is too high. Therefore, the concentration is preferably not more than 5%, more preferably not more than 1.0%, and even more preferably not more than 0.5%. On the other hand, the irregular detection-preventing effect is low when the concentration is too low. Therefore, the concentration is preferably not less than 0.005%, more preferably not less than 0.01%, and even more preferably not less than 0.05%. The range of the concentration is preferably not less than 0.005% and not more than 5%, more preferably not less than 0.001% and not more than 1.0%, and even more preferably not less than 0.05% and not more than 0.5%.

When the PEG or the like is added to an assay reagent of the present invention in advance, it may be added in such an amount that the concentration in the reaction system will fall within the above ranges.

The "weight-average molecular weight" of the PEG or the like of the present invention is measured by the following method and calculated.

Weight-average molecular weight measuring method: A sample solution (sample concentration: 1.0 g/L), which is a mixture of 20 µL of each type of aqueous PEG solution (5%) and 980 µL of tetrahydrofuran, is subjected to chromatographic analysis using a size exclusion chromatography apparatus (high-speed GPC apparatus manufactured by Tosoh Corporation: ECO SEC HLC-8320GPC, column: TSKgel Super Multiporehz-H (4.6 mm I.D. × 15 cm × 1), column temp.: 40.0°C, eluent flow rate: 0.2 mL/min, calibration curve sample: PstQuick MP-H). The chromatographic analysis is performed three times, and the average of the three measured values of weight-average molecular weight (Mw) is taken as the "weight-average molecular weight".

For PEG products used in the following experimental examples, which are manufactured by Kishida Chemical Co., Ltd., their average molecular weights announced by the manufacturer and their weight-average molecular weights determined by the above measuring method are shown below: PEG200 (product code No. 010-62905, average molecular weight 190-210, weight-average molecular weight 401); PEG400 (product code No. 010-62925, average molecular weight 380-420, weight-average molecular weight 562); PEG1000 (product code No. 010-62945, average molecular weight 950-1050, weight-average molecular weight 1033); PEG2000 (product code No. 010-62965, average molecular weight 1800-2200, weight-average molecular weight 2072); PEG4000 (product code No. 010-62975, average molecular weight 2700-3400, weight-average molecular weight 3400); PEG6000 (product code No. 010-62985, average molecular weight 7400-9000, weight-average molecular weight 10736); and PEG20000 (product code No. 020-63005, average molecular weight 18000-25000, weight-average molecular weight 23555).

The PEG or the like, which is a polymer compound, is characterized by a broad molecular weight distribution. Therefore, it is only necessary for the PEG or the like for use in the present invention that a measured value of the weight-average molecular weight falls within the range of 300 to 3000. An immunoassay reagent, containing a mixture of two or more PEGs or the like having different weight-average molecular weights, falls within the scope of the present invention if the weight-average molecular weight of the PEG mixture falls within the range of 300 to 3000. For example, when a PEG or the like having a weight-average molecular weight of 300 to 3000 is added to a reagent containing a PEG or the like having a weight-average molecular weight of not less than 2072, the reagent falls within the scope of the present invention if the weight-average molecular weight of the mixture of the PEGs or the like falls within the range of 300 to 3000.

### EXAMPLES

The following examples illustrate the present invention in greater detail and are not to be construed as limiting the scope of the invention.

### [Example 1]

### 1. Preparation of Latex Agglutination Immunoassay Reagent for Measurement of D-Dimer

### (Preparation of First Reagent)

### (1) Reagent according to the present invention

Five hundred (500) mM sodium chloride, 0.4% BSA and 0.052% ProClin 300 were dissolved in 30 mM Tris buffer (pH 8.5). PEG200 (weight-average molecular weight 401), PEG400 (weight-average molecular weight 562), PEG1000 (weight-average molecular weight 1033) and PEG2000 (weight-average molecular weight 2072) (the PEGs are manufactured by Kishida Chemical Co., Ltd.) were added to the solution in such amounts as to make the final concentrations 0.05% and 0.5%, thereby preparing Reagents 1, 2, 3 and 4 according to the present invention, respectively.

### (2) Control reagent

Five hundred (500) mM sodium chloride, 0.4% BSA and 0.052% ProClin 300 were dissolved in 30 mM Tris buffer (pH 8.5) to prepare Control Reagent 1 containing no PEG. PEG4000 (weight-average molecular weight 3400), PEG6000 (weight-average molecular weight 10736) and PEG20000 (weight-average molecular weight 23555) (the PEGs are manufactured by Kishida Chemical Co., Ltd.) were added to the Control Reagent 1 in such amounts as to make the final concentrations 0.05% and 0.5%, thereby preparing Control Reagents 2, 3 and 4, respectively.

### (Preparation of Second Reagent)

Three (3) mL of a 5% latex suspension, containing latex particles having an average particle size of 120 nm, was added to 3 mL of 20 mM Tris buffer (pH 8.5) containing 3.2 mg/mL of anti-D-dimer monoclonal antibody 03204, and the mixture was stirred at 4°C for 2 hours. Thereafter, 6 mL of 20 mM Tris buffer (pH 8.5) containing 2.0% bovine serum albumin was added to the mixture, and the mixture was stirred at 4°C for 1 hour. The resulting liquid was subjected to centrifugal separation, and the deposit was re-suspended in a 5 mM MOPS buffer (pH 7.0) in such a manner that the absorbance at a wavelength of 600 nm became 3.0 OD, thereby preparing a second reagent.

### 2. Measurement of Simultaneous Reproducibility of Latex Agglutination Immunoassay Reagent for Measurement of D-Dimer

### (1) Measurement with Hitachi 7180 automatic analyzer (reusable-type reaction cuvette, detection of transmitted light)

### (i) Measuring method

Nine point six (9.6) µL of each of calibrators having D-dimer concentrations of 0.0, 1.0, 3.0, 15, 30 and 60 µg/mL was added to 80 µL of the first reagent, and the mixture was stirred and then heated at 37°C for 5 minutes. Thereafter, 80 µL of the second reagent was added to the first reagent, and a change in the absorbance was measured at 37°C for 5 minutes at a main wavelength of 570 nm and a secondary wavelength of 800 nm. A calibration curve was created from the six measurement values of the calibrators having D-dimer concentrations of 0.0, 1.0, 3.0, 15, 30 and 60 µg/mL. The measurement was performed 10 times sequentially on D-dimer samples (sodium citrate plasma) having a known concentration of 0.33 µg/mL, and a variation coefficient was calculated, and the accuracy of each measured value and the simultaneous reproducibility were determined.

**[Table 1]**

| Automatic analyzer | Hitachi 7180 automatic analyzer | | | | | | | | | |
|---|---|---|---|---|---|---|---|---|---|---|
| Reagent Name | Cont. Rgt 1 | | Control Reagent 2 | | | Control Reagent 3 | | Control Reagent 4 | | |
| Type of PEG | None | | PEG4000 | | | PEG6000 | | PEG20000 | | |
| Weight-ave. mol. weight | --- | | 3400 | | | 10736 | | 23755 | | |
| Addition Cone. | 0% | | 0.05% | | 0.5% | 0.05% | 0.5% | 0.05% | 0.5% | |
| 10 successive measurement values (*µ*g/mL) | 0.26* | | 0.33 | | 0.26* | 0.27 | 0.27 | 0.24* | 0.19 * | |
| | 0.21* | | 0.36 | | 0.36 | 0.23* | 0.26* | 0.23* | 0.20 * | |
| | 0.17* | | 0.33 | | 0.33 | 0.28 | 0.35 | 0.31 | 0.30 | |
| | 0.19* | | 0.28 | | 0.26* | 0.28 | 0.19* | 0.22* | 0.35 | |
| | 0.18* | | 0.24* | | 0.22* | 0.32 | 0.22* | 0.21* | 0.27 | |
| | 0.32 | | 0.31 | | 0.22* | 0.30 | 0.27 | 0.25* | 0.27 | |
| | 0.29 | | 0.30 | | 0.29 | 0.28 | 0.31 | 0.24* | 0.20* | |
| | 0.24* | | 0.32 | | 0.25* | 0.22* | 0.30 | 0.35 | 0.27 | |
| | 0.31 | | 0.33 | | 0.28 | 0.35 | 0.24* | 0.32 | 0.29 | |
| | 0.33 | | 0.28 | | 0.26* | 0.28 | 0.28 | 0.30 | 0.26* | |
| Average value | 0.25 | | 0.31 | | 0.27 | 0.28 | 0.27 | 0.27 | 0.26 | |
| Standard deviation | 0.06 | | 0.03 | | 0.04 | 0.04 | 0.05 | 0.05 | 0.05 | |
| Variation coefficient (%) | 24.0%* | | 9.7% | | 14.8%* | 14.3%* | 18.5% | 18.5%* | 19.2%* | |
| | | | | | | | | | | |

| Automatic analyzer | Hitachi 7180 automatic analyzer | | | | | | | | | |
|---|---|---|---|---|---|---|---|---|---|---|
| Reagent Name | Invention Reagent 1 | | | Invention Reagent 2 | | | Invention Reagent 3 | | Invention Reagent 4 | |
| Type of PEG | PEG200 | | | PEG400 | | | PEG1000 | | PEG2000 | |
| Weight-ave. mol. weight | 401 | | | 562 | | | 1033 | | 2072 | |
| Addition Conc. | 0.05% | 0.5% | | 0,05% | | 0.5% | 0.05% | 0.5% | 0.05% | 0.5% |
| 10 successive measurement values (*µ*g/mL) | 0.39 | 0.30 | | 0.30 | | 0.31 | 0.32 | 0.31 | 0.36 | 0.32 |
| | 0.32 | 0.30 | | 0.35 | | 0.35 | 0.31 | 0.30 | 0.33 | 0.32 |
| | 0.32 | 0.37 | | 0.30 | | 0.33 | 0.28 | 0.31 | 0.36 | 0.29 |
| | 0.33 | 0.38 | | 0.30 | | 0.33 | 0.30 | 0.31 | 0.36 | 0.29 |
| | 0.35 | 0.31 | | 0.34 | | 0.33 | 0.32 | 0.31 | 0.33 | 0.31 |
| | 0.30 | 0.35 | | 0.31 | | 0.33 | 0.31 | 0.30 | 0.28 | 0,32 |
| | 0.35 | 0.31 | | 0.28 | | 0.33 | 0.30 | 0.33 | 0.31 | 0.29 |
| | 0.37 | 0.30 | | 0.30 | | 0.31 | 0.31 | 0.30 | 0.30 | 0.35 |
| | 0.33 | 0.35 | | 0.30 | | 0.32 | 0.35 | 0.29 | 0.33 | 0.34 |
| | 0.30 | 0.34 | | 0.32 | | 0.31 | 0.28 | 0.34 | 0.35 | 0.34 |
| Average value | 0.34 | 0.33 | | 0.31 | | 0.33 | 0.31 | 0.31 | 0.33 | 0.32 |
| Standard deviation | 0.02 | 0.03 | | 0.02 | | 0.01 | 0.02 | 0.02 | 0.03 | 0.02 |
| Variation coefficient (%) | 5.9% | 9.1% | | 6.5% | | 3.0% | 6.5% | 6.5% | 9.1% | 6.3% |

| | | | | | | | | | | |
|---|---|---|---|---|---|---|---|---|---|---|
| Cont. Rgt: Control Reagent Weight-ave. mol. weight: Weight-average molecular weight Addition Conc.: Addition Concentration | | | | | | | | | | |

### (ii) Measurement results

Table 1 shows the results of the measurement of simultaneous reproducibility, performed in the Hitachi 7180 automatic analyzer and using various types of PEG at different concentrations. The automatic analyzer used was of the type that uses a reusable-type reaction cuvette and detects transmitted light. In Table 1, those measured values of the D-dimer samples having the known concentration of 0.33 µg/mL, whose accuracies fall outside the range of 80 to 120% (measured values not more than 0.26 µg/mL and not less than 0.40 µg/mL), and those variation coefficient values which are not less than 10%, indicated by *.

The measurement results indicate that in the case of Control Reagent 1 containing no PEG, there were 6 measurement values whose accuracies fall outside the range of 80 to 120%, and the variation coefficient was as poor as 24.0%. In the case of Control Reagent 2 and when it contained 0.05% of PEG4000, while the variation coefficient was less than 10%, there was one measurement value whose accuracy falls outside the range of 80 to 120%. When Control Reagent 2 contained 0.5% of PEG4000, there were 6 measurement values whose accuracies fall outside the range of 80 to 120%, and the variation coefficient was as poor as 14.8%. Similarly, in the case Control Reagents 3 and 4, there were 2 to 6 measurement values whose accuracies fall outside the range of 80 to 120%, and the variation coefficients were as poor as 14.3 to 19.2%.

On the other hand, in the case of Reagents 1 to 4 of the present invention, there was no measurement value whose accuracy falls outside the range of 80 to 120%, and the variation coefficients were less than 10%. The measurement data thus verifies that the use of PEG200-2000 (weight-average molecular weight of 300 to 3000) at a concentration of 0.05 to 0.5% enhances the accuracy and the reproducibility of measurement.

### (2) Measurement with Coapresta 3000 automatic analyzer (disposable-type reaction cuvette, detection of transmitted light)

### (i) Measuring method

Twenty point zero (20.0) µL of each of calibrators having D-dimer concentrations of 0.0, 1.0, 3.0, 15, 30 and 60 µg/mL was added to 100 µL of the first reagent, and the mixture was stirred and then heated at 37°C for 5 minutes. Thereafter, 100 µL of the second reagent was added to the first reagent, and a change in the absorbance was measured at 37°C for 2 minutes at a wavelength of 570 nm. A calibration curve was created from the six measurement values of the calibrators having D-dimer concentrations of 0.0, 1.0, 3.0, 15, 30 and 60 µg/mL. The measurement was performed 10 times sequentially on a D-dimer sample (sodium citrate plasma) having a known concentration of 0.55 µg/mL, and a variation coefficient was calculated, and the simultaneous reproducibility and the accuracy of each measured value were determined.

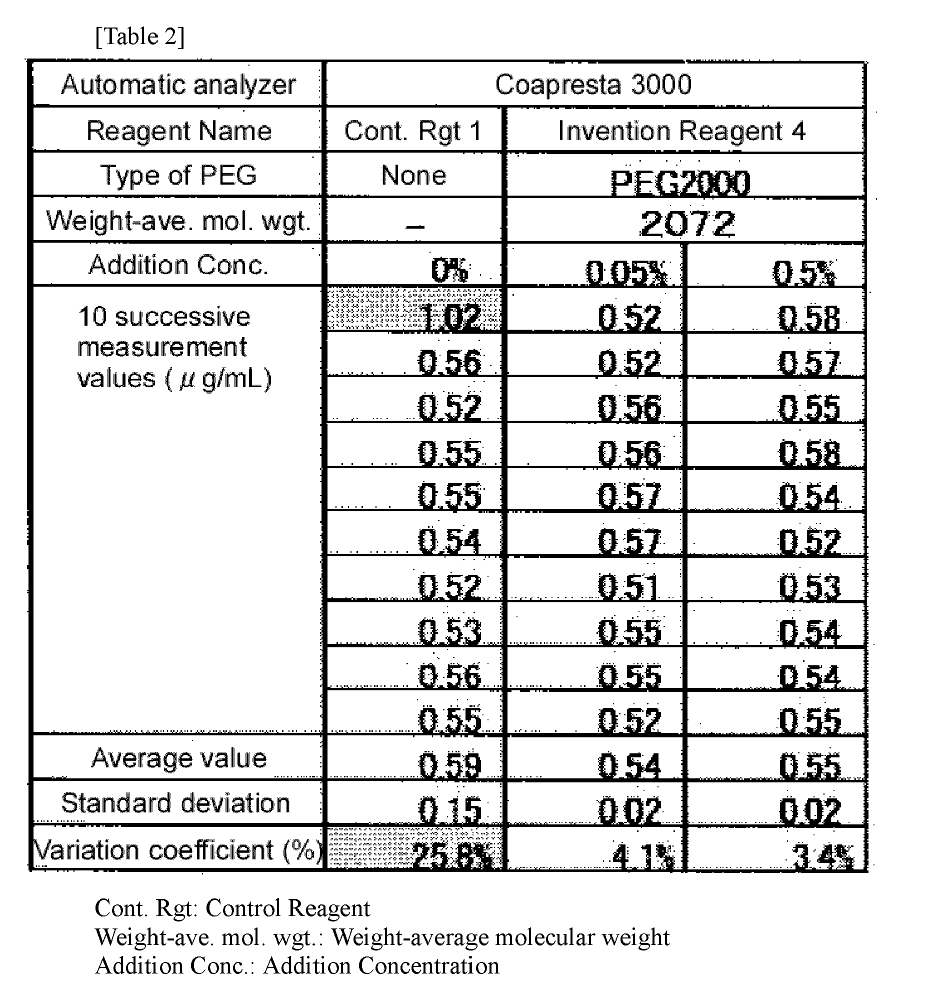

### (ii) Measurement results

Table 2 shows the results of the measurement performed in the Coapresta 3000 automatic analyzer, using PEG2000 at concentrations of 0.05% and 0.5% (Reagent 4 according to the present invention) and using Control Reagent 1. The automatic analyzer used was of the type that uses a disposable-type reaction cuvette and detects transmitted light. In Table 2, measured values of the D-dimer samples having the known concentration of 0.55 µg/mL, whose accuracy falls outside the range of 80 to 120% (measured value not more than 0.44 µg/mL or not less than 0.66 µg/mL), and a variation coefficient value which is not less than 10%, are shaded in gray. In the case of the control reagent 1, there was one measurement value whose accuracy falls outside the range of 80 to 120%, and is 185% which can be regarded as an irregular detection value. Due to the influence of this value, the variation coefficient was as poor as 25.8%.

On the other hand, in the case of the reagent 4 of the present invention, there was no measurement value whose accuracy falls outside the range of 80 to 120%, and the variation coefficients were as good as 3.4% and 4.1%.

The measurement data thus verifies that the use of PEG according to the present invention produces the effect of preventing irregularity in the detection and enhancing the reproducibility of measurement also in the case of the automatic analyzer using a disposable-type reaction cuvette.

### INDUSTRIAL APPLICABILITY

According to the present invention, by using PEG or the like having the particular weight-average molecular weight in an immunoassay reagent for use in an automatic analyzer, it becomes possible to provide an automatic analysis method which can prevent the occurrence of irregular detection and enhance the reproducibility of measurement, without causing a decrease in the measurement sensitivity, regardless of whether the reaction cuvette used is of the disposable type or the reusable type and whether the optical detection method used measures transmitted light or scattered light. It thus becomes possible to enhance the accuracy and reproducibility of immunoassay in an automatic analyzer.

## Claims

1. A method for preventing an occurrence of an irregular detection value and enhancing reproducibility of measurement upon optical detection of a reaction between an analyte in a specimen and an immunoassay reagent with an automatic analyzer, wherein the immunoassay reagent contains polyethylene glycol or the like having a weight-average molecular weight of 300 to 3000.

2. The method according to claim 1, wherein the polyethylene glycol or the like is at least one member selected from the group consisting of polyethylene glycol, polypropylene glycol, polybutylene glycol, polyoxyethylene polyoxypropylene glycol, and polyglycerol.

3. The method according to claim 1 or 2, wherein the concentration of the polyethylene glycol or the like in the reaction system is 0.05 to 0.5%.

4. The method according to any one of claims 1 to 3, wherein the polyethylene glycol or the like is polyethylene glycol.

5. The method according to any one of claims 1 to 4, wherein the immunoassay reagent is a reagent for latex turbidimetric immunoassay.

6. A method for optically detecting a reaction between an analyte in a specimen and an immunoassay reagent with an automatic analyzer, wherein the immunoassay reagent contains polyethylene glycol or the like having a weight-average molecular weight of 300 to 3000.

7. The method according to claim 6, wherein the polyethylene glycol or the like is at least one member selected from the group consisting of polyethylene glycol, polypropylene glycol, polybutylene glycol, polyoxyethylene polyoxypropylene glycol, and polyglycerol.

8. The method according to claim 6 or 7, wherein the concentration of the polyethylene glycol or the like in the reaction system is 0.05 to 0.5%.

9. The method according to any one of claims 6 to 8, wherein the polyethylene glycol or the like is polyethylene glycol.

10. The method according to any one of claims 6 to 9, wherein the immunoassay reagent is a reagent for latex turbidimetric immunoassay.

11. An immunoassay reagent for use in an optical detection method using an automatic analyzer, the reagent comprising polyethylene glycol or the like having a weight-average molecular weight of 300 to 3000.

12. The immunoassay reagent according to claim 11, wherein the polyethylene glycol or the like is at least one member selected from the group consisting of polyethylene glycol, polypropylene glycol, polybutylene glycol, polyoxyethylene polyoxypropylene glycol, and polyglycerol.

13. The immunoassay reagent according to claim 11 or 12, wherein the reagent is adjusted so that the concentration of the polyethylene glycol or the like in the reaction system becomes 0.05 to 0.5%.

14. The immunoassay reagent according to any one of claims 11 to 13, wherein the polyethylene glycol or the like is polyethylene glycol.

15. The immunoassay reagent according to any one of claims 11 to 14, wherein the immunoassay reagent is a reagent for latex turbidimetric immunoassay.

16. The immunoassay reagent according to claim 15, wherein the reagent comprises a first reagent containing a buffer, and a second reagent containing a latex, and wherein one or both of the first and second reagents contain the polyethylene glycol or the like having a weight-average molecular weight of 300 to 3000.

17. An immunoassay reagent kit comprising the immunoassay reagent according to any one of claims 11 to 16.
